# EUROPEAN PATENT APPLICATION

(11) **EP 3 886 111 A1**
(43) Date of publication of application: **29.09.2021**
(21) Application number: 20196859.1
(22) Date of filing: 18.09.2020
(51) Int. Cl.: G16H 40/00, G16H 70/20

(54) **METHOD AND APPARATUS FOR DETECTING MEDICAL CONFLICT, ELECTRONIC DEVICE AND STORAGE MEDIUM**

(30) Priority: 27.03.2020 CN 202010230814
(71) Applicant: BEIJING BAIDU NETCOM SCIENCE AND TECHNOLOGY CO. LTD., 100085 Beijing (CN)
(72) Inventor: Zheng, Yuhong, Beijing, 100085 (CN); Xue, Chengyun, Beijing, 100085 (CN); Zeng, Qifei, Beijing, 100085 (CN); Guo, Hui, Beijing, 100085 (CN); Li, Tao, Beijing, 100085 (CN)
(74) Representative: Witte, Weller & Partner Patentanwälte mbB

(57) **Abstract**

A method and apparatus for detecting a medical conflict, an electronic device are provided. A specific implementation solution includes: acquiring a medical quality control requirement of a user, and converting the medical quality control requirement into structured data corresponding thereto; detecting, through a pre-established rule-based reasoning network, whether medical entities in the structured data meet detection conditions corresponding to a leaf node, with each field in the structured data as a medical entity; in response to the medical entities in the structured data meeting the detection conditions corresponding to the leaf node, determining that the medical quality control requirement hits the leaf node; and pushing medical conflict information corresponding to the leaf node to the user.

## Description

### TECHNICAL FIELD

The present disclosure relates to the field of computer application technology, and further relates to knowledge graph technology, specifically to a method, apparatus for detecting a medical conflict, an electronic device, and a storage medium.

### BACKGROUND

Clinical decision support system (CDSS), is a medical information technology application system based on man-machine interaction, aims to provide clinical decision support for doctors and other health practitioners. By learning authoritative textbooks, pharmacopoeias and high-quality medical records of grade A tertiary hospitals, and based on Baidu medical knowledge graph, natural language processing, cognitive computing and a variety of AI technologies, it integrates probability graph reasoning, rule-based reasoning, and a multi-model decision system based on deep learning to create a clinical auxiliary decision support system that follows evidence-seeking medicine, to improve diagnosis accuracy and reduce misdiagnosis and missed diagnosis.

In the existing medical quality control technical solutions, quality control rules use independent rule fragments to restrict the relationships between medical entities. For example, P1 and P2 are two completely independent rule fragments, PI: {served drug = clarithromycin}, P2: {test: liver function test} . Through a method of inverted index, an inverted index tree is constructed for all the rule fragments in a rule set. Medical entities contained in a structured electronic medical record are matched one by one with the inverted index tree, rule fragments that meet the conditions are extracted, and finally a rule set corresponding to all the rule fragments is summarized, and a trigger reminder result corresponding to the rules is returned. It can be found from the above implementation solution of the existing technology that independent rule fragments are used to restrict the relationships between the medical entities. If there is a side relationship between medical entities, the existing technical solutions cannot use the side relationship information of the medical entities; and since the rule fragments are completely independent, a potential conflict relationship between the medical entities cannot be detected using the existing technical solutions.

### SUMMARY

In view of the above, embodiments of the present disclosure provide a method for detecting a medical conflict, an apparatus, an electronic device and a storage medium, to detect medical conflicts more comprehensively and accurately, and medical data can be expanded more conveniently for user convenience.

In a first aspect, some embodiments of the present disclosure provide a method for detecting a medical conflict, the method includes:
acquiring a medical quality control requirement of a user, and converting the medical quality control requirement into structured data corresponding thereto;
detecting, through a pre-established rule-based reasoning network, whether medical entities in the structured data meet detection conditions corresponding to a leaf node, with each field in the structured data as a medical entity;
in response to the medical entities in the structured data meeting the detection conditions corresponding to the leaf node, determining that the medical quality control requirement hits the leaf node; and
pushing medical conflict information corresponding to the leaf node to the user.

The above embodiment has following advantages and beneficial effect: the above embodiment may pre-establish a rule-based reasoning network, after converting the medical quality control requirement into the corresponding structured data, it may detect whether the medical entities in the structured data meet the detection conditions corresponding to a leaf node through the rule-based reasoning network. Therefore, it may detect whether there is the medical conflict information in the medical quality control requirement, and achieve the purpose of promptly reminding the user of a medical conflict. However, in the existing medical conflict detection methods, independent rule fragments are used to restrict the relationships between medical entities. If there is a side relationship between the medical entities, the existing technical solutions cannot use the side relationship information of the medical entities; since the rule fragments are completely independent, a potential conflict relationship between the medical entities cannot be detected using the existing technical solutions. Because embodiments of the present disclosure adopts the technical means of pre-establishing a rule-based reasoning network, it overcomes the technical problem of the inability to comprehensively and accurately detect a medical conflict in the prior art, and achieves the technical effects of more comprehensive and accurate detection of a medical conflict, more convenient expansion of medical data, and user-friendly.

In the above embodiment, the detecting, through a pre-established rule-based reasoning network, whether medical entities in the structured data meets detection conditions corresponding to a leaf node, comprises:
extracting a medical entity from the structured data as a current medical entity, flowing the current medical entity from a root node of the rule-based reasoning network to a node in a penultimate layer of nodes, and using the node in the penultimate layer of nodes, to which the current medical entity flows, as a minimum unit node hit by the current medical entity; and repeatedly performing the operation of extracting a current medical entity, until each medical entity in the structured data flows to a minimum unit node hit by the each medical entity; and
detecting whether the medical entities in the structured data meet the detection conditions corresponding to a leaf node, based on the minimum unit nodes hit by the medical entities in the structured data.

The above embodiment has following advantages and beneficial effect: the above embodiment may first flow the current medical entity from a root node of the rule-based reasoning network to a node in a penultimate layer of nodes, and then detect, based on the minimum unit nodes hit by the medical entities in the structured data, whether the medical entities in the structured data meet the detection conditions corresponding to a leaf node. The nodes in the penultimate layer of nodes in the rule-based reasoning network may store information of different medical entities respectively, so that an embodiment of the present disclosure may flow each medical entity to a node in the penultimate layer of nodes corresponding to the each medical entity, i.e., flow the each medical entity to the minimum unit node hit by the each medical entity. So that based on the minimum unit nodes hit by the medical entities in the structured data, whether the medical entities in the structured data meet the detection conditions corresponding to a leaf node is accurately detected.

In the above embodiment, the detecting whether the medical entities in the structured data meets the detection conditions corresponding to a leaf node, based on the minimum unit nodes hit by the medical entities in the structured data, comprises:
in response to the minimum unit nodes hit by the medical entities in the structured data correspond to one leaf node of the rule-based reasoning network, determining that the medical entities in the structured data meet the detection conditions corresponding to the leaf node; and
in response to the minimum unit nodes hit by the medical entities in the structured data correspond to a plurality of leaf nodes of the rule-based reasoning network, determining that medical entities in the structured data do not meet the detection conditions corresponding to a leaf node.

The above embodiment has following advantages and beneficial effect: the above embodiment may accurately judge whether the medical entities in the structured data meet the detection conditions corresponding to a leaf node by judging whether the minimum unit nodes hit by the medical entities in the structured data correspond to one leaf node of the rule-based reasoning network.

In the above embodiment, the flowing the current medical entity from the root node of the rule-based reasoning network to a node in a penultimate layer of nodes, comprises:
using the root node as the node hit by the current medical entity; and
determining, on the node hit by the current medical entity, whether the current medical entity meets a detection condition corresponding to a node in a next layer of nodes in the rule-based reasoning network; in response to the current medical entity meeting a detection condition corresponding to the node in the next layer of nodes, using the node in the next layer of nodes as the node hit by the current medical entity; and repeatedly performing the operation, until the current medical entity flows from the root node to a node in the penultimate layer of nodes.

The above embodiment has following advantages and beneficial effects: the above embodiment may determine, on the node hit by the current medical entity, whether the current medical entity meets a detection condition corresponding to a node in a next layer of nodes in the rule-based reasoning network, and thus flow the current medical entity from the root node to the node in a next layer of nodes, so that whether the medical entities in the structured data meet the detection conditions corresponding to a leaf node are accurately detected based on the minimum unit nodes hit by the medical entities in the structured data.

In the above embodiment, after the acquiring the medical quality control requirement of the user, and before the converting the medical quality control requirement into the structured data corresponding thereto, the method further comprises:
extracting a key field from the medical quality control requirement; determining a supplementary field associated with the key field based on the extracted key field;
searching medical information corresponding to the key field and medical information corresponding to the supplementary field in a pre-stored medical database; and
in response to the medical information corresponding to the key field and the medical information corresponding to the supplementary field being found in the medical database, performing the operation of converting the medical quality control requirement into the structured data corresponding thereto, based on the key field and the medical information corresponding to the key field, and the supplementary field and the medical information corresponding to the supplementary field.

The above embodiment has following advantages and beneficial effects: the above embodiment may search medical information corresponding to the key field and medical information corresponding to the supplementary field in a pre-stored medical database, and then perform the execution of the operation of converting the medical quality control requirement into the structured data corresponding thereto based on the key field and the medical information corresponding to the key field, and the supplementary field and the medical information corresponding to the supplementary field, which may ensure the completeness of the structured data.

In a second aspect, some embodiments of the present disclosure provide an apparatus for detecting a medical conflict, the apparatus includes: a conversion module, a detection module, a determination module and a pushing module; where
the conversion module, is configured to acquire a medical quality control requirement of a user, and convert the medical quality control requirement into structured data corresponding thereto;
the detection module, is configured to detect, through a pre-established rule-based reasoning network, whether medical entities in the structured data meet detection conditions corresponding to a leaf node, with each field in the structured data as a medical entity;
the determination module, is configured to determine, in response to the medical entities in the structured data meeting the detection conditions corresponding to the leaf node, that the medical quality control requirement hits the leaf node; and
the pushing module, is configured to push medical conflict information corresponding to the leaf node to the user.

In the above embodiment, the detection module comprises: a flow submodule and a detection submodule, where
the flow submodule is configured to extract a medical entity from the structured data as a current medical entity, flow the current medical entity from a root node of the rule-based reasoning network to a node in a penultimate layer of nodes, and use the node in the penultimate layer of nodes, to which the current medical entity flows, as a minimum unit node hit by the current medical entity; and repeatedly perform the operation of extracting a current medical entity, until each medical entity in the structured data flows to a minimum unit node hit by the each medical entity; and
the detection submodule is configured to detect whether the medical entities in the structured data meet the detection conditions corresponding to a leaf node, based on the minimum unit nodes hit by the medical entities in the structured data.

In the above embodiment, the detection submodule, is specifically configured to determine, in response to the minimum unit nodes hit by the medical entities in the structured data correspond to one leaf node of the rule-based reasoning network, that the medical entities in the structured data meet the detection conditions corresponding to the leaf node; and determine, in response to the minimum unit nodes hit by the medical entities in the structured data correspond to a plurality of leaf nodes of the rule-based reasoning network, that the medical entities in the structured data do not meet the detection conditions corresponding to a leaf node.

In the above embodiment, the flow submodule, is specifically configured to use the root node as the node hit by the current medical entity; and determine, on the node hit by the current medical entity, whether the current medical entity meets a detection condition corresponding to a node in a next layer of nodes in the rule-based reasoning network; in response to the current medical entity meeting a detection condition corresponding to the node in the next layer of nodes, use the node in the next layer of nodes as the node hit by the current medical entity; and repeatedly perform the operation, until the current medical entity flows from the root node to a node in the penultimate layer of nodes.

In the above embodiment, the conversion module, is further configured to extract a key field from the medical quality control requirement; determine a supplementary field associated with the key field based on the extracted key field; search medical information corresponding to the key field and medical information corresponding to the supplementary field in a pre-stored medical database; and perform the operation of converting the medical quality control, in response to the medical information corresponding to the key field and the medical information corresponding to the supplementary field being found in the medical database, requirement into corresponding structured data, based on the key field and the medical information corresponding to the key field, and the supplementary field and the medical information corresponding to the supplementary field.

In a third aspect, some embodiments of the present disclosure provide an electronic device, the electronic device includes:
at least one processor; and
a memory, communicatively connected to the at least one processor; where the memory, storing instructions executable by the at least one processor, the instructions, when executed by the at least one processor, cause the at least one processor to perform the method according to any one of the embodiments of the present disclosure.

In a fourth aspect, some embodiments of the present disclosure provide a non-transitory computer readable storage medium, storing computer instructions, the computer instructions, being used to cause a computer to perform the method according to any one of the embodiments of the present disclosure.

An embodiment of the present disclosure has following advantages and beneficial effect: the above embodiment may pre-establish a rule-based reasoning network, after converting the medical quality control requirement into the corresponding structured data, it may detect whether the medical entities in the structured data meet the detection conditions corresponding to a leaf node through the rule-based reasoning network. Therefore, it may detect whether there is the medical conflict information in the medical quality control requirement, and achieve the purpose of promptly reminding the user of a medical conflict. However, in the existing medical conflict detection methods, independent rule fragments are used to restrict the relationships between medical entities. If there is a side relationship between the medical entities, the existing technical solutions cannot use the side relationship information of the medical entities; since the rule fragments are completely independent, a potential conflict relationship between the medical entities cannot be detected using the existing technical solutions. Because the present disclosure adopts the technical means of pre-establishing a rule-based reasoning network, it overcomes the technical problem of the inability to comprehensively and accurately detect a medical conflict in the prior art, and achieves the technical effects of more comprehensive and accurate detection of a medical conflict, more convenient expansion of medical data, and user-friendly; moreover, the technical solution of embodiments of the present disclosure is simple and convenient to implement, easy to popularize, and have a wider application range.

The other effects of the above-mentioned optional methods will be described below in conjunction with specific embodiments.

### BRIEF DESCRIPTION OF THE DRAWINGS

Accompanying drawings are used to better understand the present solution and do not constitute a limitation to the present disclosure, in which:
Fig. 1 is a schematic flowchart of a method for detecting a medical conflict provided by Embodiment 1 of the present disclosure;
Fig. 2 is a schematic flowchart of a method for detecting a medical conflict provided by Embodiment 2 of the present disclosure;
Fig. 3 is a schematic diagram of a first structure of a rule-based reasoning network provided by Embodiment 2 of the present disclosure;
Fig. 4 is a schematic diagram of a second structure of a rule-based reasoning network provided by Embodiment 2 of the present disclosure;
Fig. 5 is a schematic structural diagram of an apparatus for detecting a medical conflict provided by Embodiment 3 of the present disclosure;
Fig. 6 is a schematic structural diagram of a detection module provided by Embodiment 3 of the present disclosure; and
Fig. 7 is a block diagram of an electronic device for implementing the method for detecting a medical conflict of embodiments of the present disclosure.

### DETAILED DESCRIPTION OF EMBODIMENTS

The following describes exemplary embodiments of the present disclosure in conjunction with the accompanying drawings, which includes various details of embodiments of the present disclosure to facilitate understanding, and they should be considered as merely exemplary. Therefore, those of ordinary skill in the art should recognize that various changes and modifications may be made to the embodiments described herein without departing from the scope of the present disclosure. Also, for clarity and conciseness, descriptions of well-known functions and structures are omitted in the following description.

### Embodiment 1

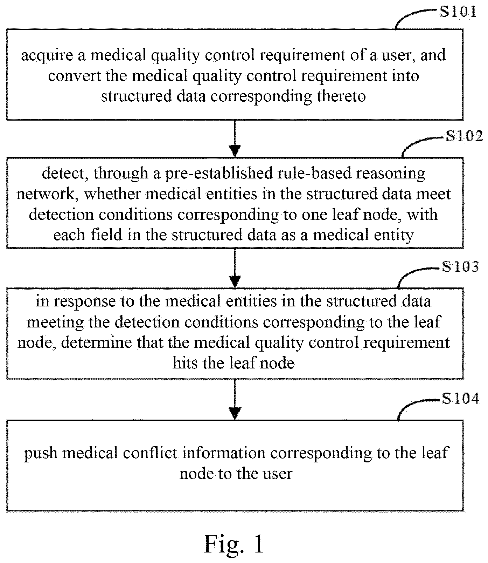

Fig. 1 is a schematic flowchart of a method for detecting a medical conflict provided by Embodiment 1 of the present disclosure. The method may be performed by an apparatus for detecting a medical conflict or an electronic device. The apparatus or the electronic device may be implemented by software and/or hardware. The apparatus or electronic device may be integrated in any smart device having a network communication function. As shown in Fig. 1, the method for detecting a medical conflict may include the following steps:
S101, acquiring a medical quality control requirement of a user, and converting the medical quality control requirement into structured data corresponding thereto.

In a specific embodiment of the present disclosure, the electronic device may acquire the medical quality control requirement of the user, and convert the medical quality control requirement into structured data corresponding thereto. Specifically, the medical quality control requirement may refer to medical information that the user wants to detect whether there is a medical conflict. For example, the medical quality control requirement may be a prescription, a laboratory test sheet, or an inspection sheet issued by a doctor to a patient. The medical conflict refers to contradictory or wrong information in the medical quality control requirement. For example, for a certain kind of medicine for children which is prescribed that it can be taken only by those over three years old, if prescription prescribed by doctor to a two-year-old child includes this certain kind of medicine, the prescription is a prescription with medical conflict.

In this step, the electronic device may use a natural language understanding (NLU) model to transform the medical quality control requirement into the structured data corresponding thereto. In this step, the electronic device may input the medical quality control requirement of the user into the NLU model, and the NLU model may output the structured data corresponding to the medical quality control requirement. Natural language processing (NLP) is a technology that uses natural language to communicate with a computer. Because the key to processing natural language is to make the computer "understand" natural language, natural language processing is also called natural language understanding, also known as computational linguistics. On the one hand, it is a branch of language information processing; on the other hand, it is one of the core topics of artificial intelligence (AI).

Preferably, in a specific embodiment of the present disclosure, after acquiring the medical quality control requirement of the user, before converting the medical quality control requirement into the corresponding structured data, the electronic device may also extract a key field from the medical quality control requirement; determine a supplementary field associated with the key field based on the extracted key field; then search medical information corresponding to the key field and medical information corresponding to the supplementary field in a pre-stored medical database; and if the medical information corresponding to the key field and the medical information corresponding to the supplementary field are found in the medical database, perform the operation of converting the medical quality control requirement into corresponding structured data, based on the key field and the medical information corresponding to the key field, and the supplementary field and the medical information corresponding to the supplementary field.

S102, detecting, through a pre-established rule-based reasoning network, whether medical entities in the structured data meet detection conditions corresponding to one leaf node, with each field in the structured data as a medical entity.

In a specific embodiment of the present disclosure, the electronic device may detect, through a pre-established rule-based reasoning network, whether the medical entities in the structured data meet the detection conditions corresponding to one leaf node, with each field in the structured data as a medical entity. For example, assuming that the structured data corresponding to the medical quality control requirement of the user includes three fields, namely: field 1, field 2, and field 3. In this step, the electronic device may use field 1 as medical entity 1, field 2 as medical entity 2, and field 3 as medical entity 3. Assuming that a leaf node in the rule-based reasoning network corresponds to three detection conditions, namely: detection condition 1, detection condition 2 and detection condition 3. If medical entity 1 meets detection condition 1 corresponding to the leaf node, medical entity 2 meets detection condition 2 corresponding to the leaf node, and medical entity 3 meets detection condition 3 corresponding to the leaf node, the electronic device may determine that the structured data meets detection conditions corresponding to the leaf node.

S103, in response to the medical entities in the structured data meeting the detection conditions corresponding to the leaf node, determining that the medical quality control requirement hits the leaf node.

In a specific embodiment of the present disclosure, if medical entities in the structured data meet the detection conditions corresponding to a leaf node, the electronic device may determine that the medical quality control requirement hits the leaf node. For example, assuming that the structured data corresponding to the medical quality control requirement of the user includes three fields, namely: field 1, field 2, and field 3. In this step, the electronic device may use field 1 as medical entity 1, field 2 as medical entity 2, and field 3 as medical entity 3. Assuming that a leaf node in the rule-based reasoning network corresponds to three detection conditions, namely: detection condition 1, detection condition 2 and detection condition 3. If medical entity 1 meets detection condition 1 corresponding to the leaf node, medical entity 2 meets detection condition 2 corresponding to the leaf node, and medical entity 3 meets detection condition 3 corresponding to the leaf node, the electronic device may determine that the medical quality control requirement hits the leaf node. In addition, if the medical entities in the structured data does not meet the detection conditions corresponding to a leaf node, the electronic device may determine that the medical quality control requirement does not hit the leaf node. For example, if medical entity 1 meets detection condition 1 corresponding to the leaf node, medical entity 2 meets detection condition 2 corresponding to the leaf node, and medical entity 3 does not meet detection condition 3 corresponding to the leaf node, the electronic device may determine that the medical quality control requirement does not hit the leaf node.

S104, pushing medical conflict information corresponding to the leaf node to the user.

In a specific embodiment of the present disclosure, the electronic device may push the medical conflict information corresponding to the leaf node to the user. Specifically, each leaf node in the rule-based reasoning network corresponds to a piece of medical conflict information. If the medical quality control requirement of the user hits a certain leaf node, it means that the medical quality control requirement of the user has medical conflict information corresponding to the certain leaf node. Therefore, the electronic device may push the medical conflict information corresponding to the certain leaf node to the user, and promptly remind the user of the medical conflict information in the medical quality control requirement.

The method for detecting a medical conflict proposed in embodiments of the present disclosure, first acquires a medical quality control requirement of a user, and converts the medical quality control requirement into corresponding structured data; then detects, through a pre-established rule-based reasoning network, whether medical entities in the structured data meet detection conditions corresponding to a leaf node, with each field in the structured data as a medical entity; in response to the medical entities in the structured data meeting the detection conditions corresponding to the leaf node, determines that the medical quality control requirement hits the leaf node; and pushes medical conflict information corresponding to the leaf node to the user. That is, the present disclosure may pre-establish a rule-based reasoning network. After converting the medical quality control requirement into structured data corresponding thereto, it may detect whether medical entities in the structured data meet detection conditions corresponding to a leaf node through the rule-based reasoning network. Therefore, it may detect whether there is the medical conflict information in the medical quality control requirement, and achieve the purpose of promptly reminding the user of a medical conflict. However, in the existing medical conflict detection methods, independent rule fragments are used to restrict the relationships between medical entities. If there is a side relationship between the medical entities, the existing technical solutions cannot use the side relationship information of the medical entities; since the rule fragments are completely independent, a potential conflict relationship between the medical entities cannot be detected using the existing technical solutions. Because embodiments of the present disclosure adopt the technical means of pre-establishing a rule-based reasoning network, it overcomes the technical problem in the prior art, i.e., the inability to comprehensively and accurately detect a medical conflict, and achieves the technical effects of more comprehensive and accurate detection of a medical conflict, more convenient expansion of medical data, and user-friendly; moreover, the technical solution of embodiments of the present disclosure is simple and convenient to implement, easy to popularize, and have a wider application range.

### Embodiment 2

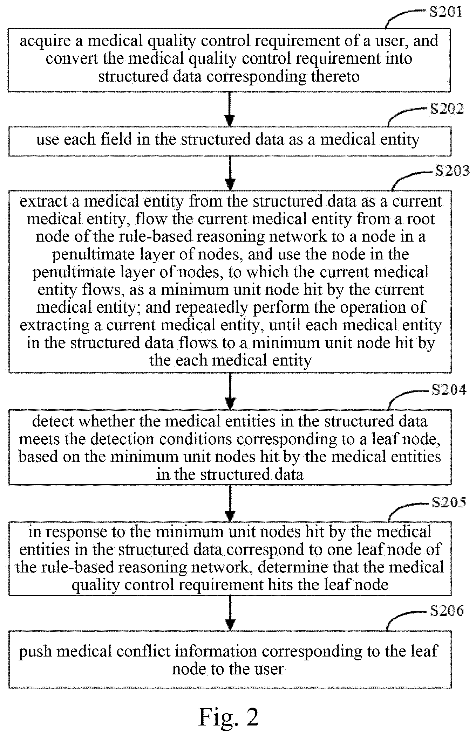

Fig. 2 is a schematic flowchart of a method for detecting a medical conflict provided by Embodiment 2 of the present disclosure. As shown in Fig. 2, the method for detecting a medical conflict may include the following steps:

S201, acquiring a medical quality control requirement of a user, and converting the medical quality control requirement into structured data corresponding thereto.

In a specific embodiment of the present disclosure, the electronic device may acquire the medical quality control requirement of the user, and convert the medical quality control requirement into the structured data corresponding thereto. Specifically, the electronic device may convert the medical quality control requirement into the corresponding structured data through an NLU model. In this step, the electronic device may input the medical quality control requirement of the user into the NLU model, and the NLU model may output the structured data corresponding to the medical quality control requirement.

S202, using each field in the structured data as a medical entity.

In a specific embodiment of the present disclosure, the electronic device may use each field in the structured data as a medical entity. For example, assuming that the structured data corresponding to the medical quality control requirement of the user includes three fields, namely: field 1, field 2, and field 3. In this step, the electronic device may use field 1 as medical entity 1, field 2 as medical entity 2, and field 3 as medical entity 3.

S203, extracting a medical entity from the structured data as a current medical entity, flowing the current medical entity from a root node of the rule-based reasoning network to a node in a penultimate layer of nodes, and using the node in the penultimate layer of nodes to which the current medical entity flows as a minimum unit node hit by the current medical entity; and repeatedly performing the operation of extracting a current medical entity, until each medical entity in the structured data flows to a minimum unit node hit by the each medical entity.

In a specific embodiment of the present disclosure, the electronic device may extract a medical entity from the structured data as the current medical entity, flow the current medical entity from the root node of the rule-based reasoning network to a node in the penultimate layer of nodes, and use the node in the penultimate layer of nodes, to which the current medical entity flows, as the minimum unit node hit by the current medical entity; and repeatedly perform the operation of extracting a current medical entity, until each medical entity in the structured data flows to the minimum unit node hit by the each medical entity. Specifically, assuming that the structured data corresponding to the medical quality control requirement of the user includes three medical entities, namely: medical entity 1, medical entity 2, and medical entity 3; in this step, the electronic device first uses medical entity 1 as the current medical entity, flows medical entity 1 from the root node of the rule-based reasoning network to a node in the penultimate layer of nodes, and uses the node in the penultimate layer of nodes, to which medical entity 1 flows, as the minimum unit node hit by medical entity 1; and repeatedly performs the operation of extracting a current medical entity, then uses medical entity 2 as the current medical entity, flows medical entity 2 from the root node of the rule-based reasoning network to a node in the penultimate layer of nodes, and uses the node in the penultimate layer of nodes, to which medical entity 2 flows, as the minimum unit node hit by medical entity 2; and repeatedly performs the operation of extracting a current medical entity, and uses medical entity 3 as the current medical entity, flows medical entity 3 from the root node of the rule-based reasoning network to a node in the penultimate layer of nodes, and uses the node in the penultimate layer of nodes, to which medical entity 3 flows, as the minimum unit node hit by medical entity 3.

In a specific embodiment of the present disclosure, during flowing the current medical entity from the root node of the rule-based reasoning network to a node in the penultimate layer of nodes, the electronic device may first use the root node as a node hit by the current medical entity; then determine, on the node hit by the current medical entity, whether the current medical entity meets a detection condition corresponding to any node in a next layer of nodes in the rule-based reasoning network; if the current medical entity meets a detection condition corresponding to any node in the next layer of nodes, use the node in the next layer of nodes as the node hit by the current medical entity; and repeatedly perform the operation, until the current medical entity flows from the root node to a node in the penultimate layer of nodes.

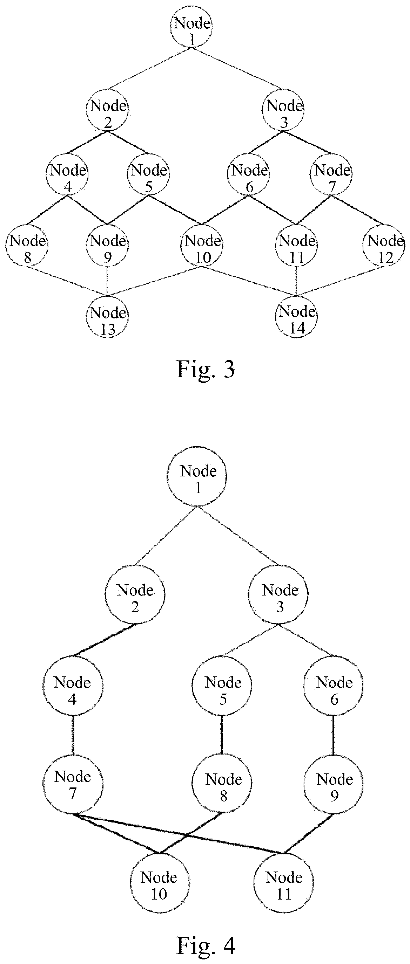

Fig. 3 is a first structural schematic diagram of a rule-based reasoning network provided by Embodiment 2 of the present disclosure. As shown in Fig. 3, the rule-based reasoning network includes five layers of nodes, namely: first layer of nodes, second layer of nodes, third layer of nodes, fourth layer of nodes, and fifth layer of nodes; the first layer of nodes include: node 1, node 1 is the root node of the rule-based reasoning network; the second layer of nodes include: node 2 and node 3; the third layer of nodes include: node 4, node 5, node 6, and node 7; the fourth layer of nodes include: node 8, node 9, node 10, node 11 and node 12; and the fifth layer of nodes include: node 13 and node 14; node 13 and node 14 are two leaf nodes of the rule-based reasoning network. Specifically, node 1 connects node 2 and node 3 respectively, indicating that a medical entity may flow from the root node to node 2 or from the root node to node 3; node 2 connects node 4 and node 5 respectively, indicating that a medical entity may flow from node 2 to node 4 or from node 2 to node 5; node 3 connects node 6 and node 7 respectively, indicating that a medical entity may flow from node 3 to node 6 or from node 3 to node 7; and so on. Each node may save its own corresponding detection condition. For example, when a certain medical entity flows from the first layer of nodes to the second layer of nodes, if the medical entity meets a detection condition corresponding to node 2, the medical entity may flow to node 2; or if the medical entity meets a detection condition corresponding to node 3, the medical entity may flow to node 3.

Fig. 4 is a second structural schematic diagram of a rule-based reasoning network provided by Embodiment 2 of the present disclosure. As shown in Fig. 4, for the given rules 1 and 2, rule 1: Person(is_vip=true, age>18)&&Product(name="Marlboro")=>Action(discount=80%) ; rule 2: Person(is_vip=true, age>18)&&Product(name="Double happiness")=>Action(discount=75%), the rule-based reasoning network shown below may be generated. The rule-based reasoning network may include five layers of nodes, namely: first layer of nodes, second layer of nodes, third layer of nodes, fourth layer of nodes, and fifth layer of nodes; the first layer of nodes include: node 1, node 1 is the root node of the rule-based reasoning network; the second layer of nodes include: node 2 and node 3; the third layer of nodes include: node 4, node 5, and node 6; the fourth layer of nodes include: node 7, node 8, and node 9; and the fifth layer of nodes include: node 10 and node 11; node 10 and node 11 are two leaf nodes of the rule-based reasoning network. Specifically, node 1 connects node 2 and node 3 respectively, indicating that a medical entity may flow from the root node to node 2 or from the root node to node 3; node 2 connects node 4, indicating that a medical entity may flow from node 2 to node 4; node 3 connects node 5 and node 6 respectively, indicating that a medical entity may flow from node 3 to node 5 or from node 3 to node 6; and so on. Specifically, the detection condition corresponding to node 1 is null; the detection condition corresponding to node 2 is: person=true, indicating that the person (user) field in the structured data is a true value; the detection condition corresponding to node 3 is: product=true, indicating that the product (product) field in the structured data is a true value; the detection condition corresponding to node 4 is: person (is_vip=true), indicating that the value of is_vip (vip user) in the person field is a true value; the detection condition corresponding to node 5 is: product (name="Marlboro"), indicating that the name (name) in the product field is Marlboro; the detection condition corresponding to node 6 is: product (name="Double happiness"), indicating that the name (name) in the product field is Double happiness; the detection condition corresponding to node 7 is: person (is_vip=true, age>18), indicating that the value of is_vip in the person field is a true value and age in the person field is greater than 18 years old; the detection condition corresponding to node 8 is: product (name="Marlboro"); the detection condition corresponding to node 9 is: product (name="Double happiness"); the detection condition corresponding to node 10 is: person(is_vip=true, age>18)&&product(name="Marlboro"), indicating that the value of is_vip in the person field is a true value and age in the person field is greater than 18 years old, and the name (name) in the product field is Marlboro; and the detection condition corresponding to node 11 is: person (is_vip=true, age>18) && product (name="Double happiness"), indicating that the value of is_vip in the person field is a true value and age in the person field is greater than 18 years old, and the name (name) in the product field is Double happiness. Preferably, when a certain medical entity hits a leaf node of the rule-based reasoning network, the medical entity may also flow to a behavior node connected to the leaf node; each behavior node may save its own execution condition. As shown in Fig. 4, when a certain medical entity flows to node 10, it may also flows to a behavior node connected to node 10. The execution condition of the behavior node is: discount=80%, indicating that executing an 80% discount; and when a certain medical entity flows to node 11, the medical entity may also flow to a behavior node connected to node 11. The execution condition of the behavior node is: discount=75%, indicating that executing a 75% discount.

S204, detecting whether the medical entities in the structured data meets the detection conditions corresponding to a leaf node, based on the minimum unit nodes hit by the medical entities in the structured data.

In a specific embodiment of the present disclosure, the electronic device may detect whether medical entities in the structured data meet detection conditions corresponding to a leaf node, based on the minimum unit nodes hit by medical entities in the structured data. Specifically, if the minimum unit nodes hit by the medical entities in the structured data corresponds to a leaf node in the rule-based reasoning network, the electronic device may determine that the medical entities in the structured data meet detection conditions corresponding to the leaf node; or if the minimum unit nodes hit by the medical entities in the structured data correspond to a plurality of leaf nodes in the rule-based reasoning network, the electronic device may determine that medical entities in the structured data do not meet the detection conditions corresponding to a leaf node.

S205, in response to the minimum unit nodes hit by the medical entities in the structured data correspond to one leaf node of the rule-based reasoning network, determining that the medical quality control requirement hits the leaf node.

In a specific embodiment of the present disclosure, if the medical entities in the structured data meet the detection conditions corresponding to a leaf node, the electronic device may determine that the medical quality control requirement hits the leaf node. For example, assuming that the structured data corresponding to the medical quality control requirement of the user includes three fields, namely: field 1, field 2, and field 3. In this step, the electronic device may use field 1 as medical entity 1, field 2 as medical entity 2, and field 3 as medical entity 3. Assuming that a leaf node in the rule-based reasoning network corresponds to three detection conditions, namely: detection condition 1, detection condition 2 and detection condition 3. If medical entity 1 meets detection condition 1 corresponding to the leaf node, medical entity 2 meets detection condition 2 corresponding to the leaf node, and medical entity 3 meets detection condition 3 corresponding to the leaf node, the electronic device may determine that the medical quality control requirement hits the leaf node. In addition, if the medical entities in the structured data do not meet the detection conditions corresponding to a leaf node, the electronic device may determine that the medical quality control requirement does not hit the leaf node. For example, if medical entity 1 meets detection condition 1 corresponding to the leaf node, medical entity 2 meets detection condition 2 corresponding to the leaf node, and medical entity 3 does not meet detection condition 3 corresponding to the leaf node, the electronic device may determine that the medical quality control requirement does not hit the leaf node.

S206, pushing medical conflict information corresponding to the leaf node to the user.

In a specific embodiment of the present disclosure, the electronic device may push the medical conflict information corresponding to the leaf node to the user. Specifically, each leaf node in the rule-based reasoning network corresponds to a piece of medical conflict information. If the medical quality control requirement of the user hits a certain leaf node, it means that the medical quality control requirement of the user has medical conflict information corresponding to the leaf node. Therefore, the electronic device may push the piece of medical conflict information corresponding to the leaf node to the user, and promptly remind the user of the medical conflict information in the medical quality control requirement.

Preferably, in specific embodiments of the present disclosure, after acquiring the medical quality control requirement of the user, before converting the medical quality control requirement into the corresponding structured data, the electronic device may also extract a key field from the medical quality control requirement; determine a supplementary field associated with the key field based on the extracted key field; then search medical information corresponding to the key field and medical information corresponding to the supplementary field in a pre-stored medical database; and if the medical information corresponding to the key field and the medical information corresponding to the supplementary field are found in the medical database, perform the operation of converting the medical quality control requirement into corresponding structured data, based on the key field and the medical information corresponding to the key field, and the supplementary field and the medical information corresponding to the supplementary field.

The method for detecting a medical conflict proposed in embodiments of the present disclosure, first acquires a medical quality control requirement of a user, and converts the medical quality control requirement into corresponding structured data; then detects whether the medical entities in the structured data meet the detection conditions corresponding to a leaf node through a pre-established rule-based reasoning network, with each field in the structured data as a medical entity; in response to the medical entities in the structured data meeting the detection conditions corresponding to the leaf node, determines that the medical quality control requirement hits the leaf node; and pushes medical conflict information corresponding to the leaf node to the user. That is, an embodiment of the present disclosure may pre-establish a rule-based reasoning network. After converting the medical quality control requirement into the corresponding structured data, it may detect whether the medical entities in the structured data meet the detection conditions corresponding to a leaf node through the rule-based reasoning network. Therefore, it may detect whether there is the medical conflict information in the medical quality control requirement, and achieve the purpose of promptly reminding the user of a medical conflict. However, in the existing medical conflict detection methods, independent rule fragments are used to restrict the relationships between medical entities. If there is a side relationship between the medical entities, the existing technical solutions cannot use the side relationships information of the medical entities; since the rule fragments are completely independent, a potential conflict relationship between the medical entities cannot be detected using the existing technical solutions. Because an embodiment of the present disclosure adopts the technical means of pre-establishing a rule-based reasoning network, it overcomes the technical problem of the inability to comprehensively and accurately detect a medical conflict in the prior art, and achieves the technical effects of more comprehensive and accurate detection of a medical conflict, more convenient expansion of medical data, and user-friendly; moreover, the technical solution of embodiments of the present disclosure is simple and convenient to implement, easy to popularize, and have a wider application range.

### Embodiment 3

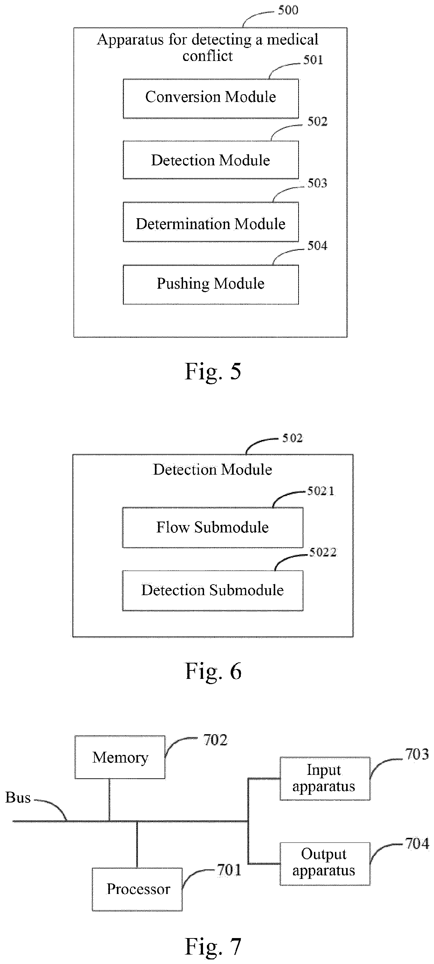

Fig. 5 is a schematic structural diagram of an apparatus for detecting a medical conflict provided by Embodiment 3 of the present disclosure. As shown in Fig. 5, the apparatus 500 includes: a conversion module 501, a detection module 502, a determination module 503 and a pushing module 504; where,
the conversion module 501, is configured to acquire a medical quality control requirement of a user, and convert the medical quality control requirement into structured data corresponding thereto;
the detection module 502, is configured to detect, through a pre-established rule-based reasoning network, whether medical entities in the structured data meet detection conditions corresponding to a leaf node, with each field in the structured data as a medical entity;
the determination module 503, is configured to determine, in response to the medical entities in the structured data meeting the detection conditions corresponding to the leaf node, that the medical quality control requirement hits the leaf node; and
the pushing module 504, is configured to push medical conflict information corresponding to the leaf node to the user.

Fig. 6 is a schematic structural diagram of a detection module provided by Embodiment 3 of the present disclosure. As shown in Fig. 6, the detection module 502 includes: a flow submodule 5021 and a detection submodule 5022; where,
the flow submodule 5021, is configured to extract a medical entity from the structured data as a current medical entity, flow the current medical entity from a root node of the rule-based reasoning network to a node in a penultimate layer of nodes, and use the node in the penultimate layer of nodes, to which the current medical entity flows, as a minimum unit node hit by a current medical entity; and repeatedly perform the operation of extracting a current medical entity, until each medical entity in the structured data flows to a minimum unit node hit by the each medical entity; and
the detection submodule 5022, is configured to detect whether the medical entities in the structured data meet the detection conditions corresponding to a leaf node, based on the minimum unit nodes hit by the medical entities in the structured data.

Further, the detection submodule 5022, is specifically configured to determine, in response to the minimum unit nodes hit by the medical entities in the structured data correspond to one leaf node of the rule-based reasoning network, that the medical entities in the structured data meet the detection conditions corresponding to the leaf node; and determine, in response to the minimum unit nodes hit by the medical entities in the structured data correspond to a plurality of leaf nodes of the rule-based reasoning network, that the medical entities in the structured data do not meet the detection conditions corresponding to a leaf node.

Further, the flow submodule 5021, is specifically configured to use the root node as the node hit by the current medical entity; and determine, on the node hit by the current medical entity, whether the current medical entity meets a detection condition corresponding to a node in a next layer of nodes in the rule-based reasoning network; in response to the current medical entity meeting a detection condition corresponding to the node in the next layer of nodes, use the node in the next layer of nodes as the node hit by the current medical entity; and repeatedly perform the operation, until the current medical entity flows from the root node to a node in the penultimate layer of nodes.

Further, the conversion module 501, is further configured to extract a key field from the medical quality control requirement; determine a supplementary field associated with the key field based on the extracted key field; search medical information corresponding to the key field and medical information corresponding to the supplementary field in a pre-stored medical database; and perform the operation of converting the medical quality control, in response to the medical information corresponding to the key field and the medical information corresponding to the supplementary field being found in the medical database, requirement into corresponding structured data, based on the key field and the medical information corresponding to the key field, and the supplementary field and the medical information corresponding to the supplementary field.

The apparatus for detecting a medical conflict may perform the method provided in any embodiment of the present disclosure, and has corresponding functional modules and beneficial effects for performing the method. For technical details not described in detail in the present embodiment, reference may be made to the method for detecting a medical conflict provided in any embodiment of the present disclosure.

### Embodiment 4

According to an embodiment of the present disclosure, the present disclosure also provides an electronic device and a readable storage medium.

As shown in Fig. 7, is a block diagram of an electronic device of the method for detecting a medical conflict according to the embodiments of the present disclosure. The electronic device is intended to represent various forms of digital computers, such as laptop computers, desktop computers, workbenches, personal digital assistants, servers, blade servers, mainframe computers, and other suitable computers. The electronic device may also represent various forms of mobile apparatuses, such as personal digital processors, cellular phones, smart phones, wearable devices, and other similar computing apparatuses. The components shown herein, their connections and relationships, and their functions are merely examples, and are not intended to limit the implementation of the present disclosure described and/or claimed herein.

As shown in Fig 7, the electronic device includes: one or more processors 701, a memory 702, and interfaces for connecting various components, including high-speed interfaces and low-speed interfaces. The various components are connected to each other using different buses, and may be installed on a common motherboard or in other methods as needed. The processor may process instructions executed within the electronic device, including instructions stored in or on the memory to display graphic information of GUI on an external input/output apparatus (such as a display device coupled to the interface). In other embodiments, a plurality of processors and/or a plurality of buses may be used together with a plurality of memories and a plurality of memories if desired. Similarly, a plurality of electronic devices may be connected, and the devices provide some necessary operations (for example, as a server array, a set of blade servers, or a multi-processor system). In Fig. 7, one processor 701 is used as an example.

The memory 702 is a non-transitory computer readable storage medium provided by some embodiments of the present disclosure. The memory stores instructions executable by at least one processor, so that the at least one processor performs the method for detecting a medical conflict provided by some embodiments of the present disclosure. The non-transitory computer readable storage medium of the present disclosure stores computer instructions for causing a computer to perform the method for detecting a medical conflict provided by some embodiments of the present disclosure.

The memory 702, as a non-transitory computer readable storage medium, may be used to store non-transitory software programs, non-transitory computer executable programs and modules, such as program instructions/modules corresponding to the method for detecting a medical conflict in embodiments of the present disclosure (for example, the conversion module 501, the detection module 502, the determination module 503 and the pushing module 504 shown in Fig. 5) . The processor 701 executes the non-transitory software programs, instructions, and modules stored in the memory 702 to execute various function applications and data processing of the server, that is, to implement the method for detecting a medical conflict in the foregoing method embodiments.

The memory 702 may include a storage program area and a storage data area, where the storage program area may store an operating system and at least one function required application program; and the storage data area may store data created by the use of the electronic device according to the method for detecting a medical conflict, etc. In addition, the memory 702 may include a high-speed random access memory, and may also include a non-transitory memory, such as at least one magnetic disk storage device, a flash memory device, or other non-transitory solid-state storage devices. In some embodiments, the memory 702 may optionally include memories remotely provided with respect to the processor 70, and these remote memories may be connected to the electronic device of the method for detecting a medical conflict through a network. Examples of the above network include but are not limited to the Internet, intranet, local area network, mobile communication network, and combinations thereof.

The electronic device of the method for detecting a medical conflict may further include: an input apparatus 703 and an output apparatus 704. The processor 701, the memory 702, the input apparatus 703, and the output apparatus 704 may be connected through a bus or in other methods. In Fig. 7, connection through a bus is used as an example.

The input apparatus 703 may receive input digital or character information, and generate key signal inputs related to user settings and function control of the electronic device of the method for detecting a medical conflict, such as touch screen, keypad, mouse, trackpad, touchpad, pointing stick, one or more mouse buttons, trackball, joystick and other input apparatuses. The output apparatus 704 may include a display device, an auxiliary lighting apparatus (for example, LED), a tactile feedback apparatus (for example, a vibration motor), and the like. The display device may include, but is not limited to, a liquid crystal display (LCD), a light emitting diode (LED) display, and a plasma display. In some embodiments, the display device may be a touch screen.

Various embodiments of the systems and technologies described herein may be implemented in digital electronic circuit systems, integrated circuit systems, dedicated ASICs (application specific integrated circuits), computer hardware, firmware, software, and/or combinations thereof. These various embodiments may include: being implemented in one or more computer programs that can be executed and/or interpreted on a programmable system that includes at least one programmable processor. The programmable processor may be a dedicated or general-purpose programmable processor, and may receive instructions and data from a storage system, at least one input apparatus, and at least one output apparatus, and transmit the data and instructions to the storage system, the at least one input apparatus, and the at least one output apparatus.

These computing programs (also referred to as programs, software, software applications, or codes) include machine instructions of the programmable processor and may use high-level processes and/or object-oriented programming languages, and/or assembly/machine languages to implement these computing programs. As used herein, the terms "machine readable medium" and "computer readable medium" refer to any computer program product, device, and/or apparatus (for example, magnetic disk, optical disk, memory, programmable logic apparatus (PLD)) used to provide the machine instructions and/or data to the programmable processor, including machine readable medium that receives the machine instructions as machine readable signals. The term "machine readable signal" refers to any signal used to provide the machine instructions and/or data to the programmable processor.

In order to provide interaction with a user, the systems and technologies described herein may be implemented on a computer, and the computer has: a display apparatus for displaying information to the user (for example, CRT (cathode ray tube) or LCD (liquid crystal display) monitor); and a keyboard and a pointing apparatus (for example, mouse or trackball), and the user may use the keyboard and the pointing apparatus to provide input to the computer. Other types of apparatuses may also be used to provide interaction with the user; for example, feedback provided to the user may be any form of sensory feedback (for example, visual feedback, auditory feedback, or tactile feedback); and any form (including acoustic input, voice input, or tactile input) may be used to receive input from the user.

The systems and technologies described herein may be implemented in a computing system that includes backend components (e.g., as a data server), or a computing system that includes middleware components (e.g., application server), or a computing system that includes frontend components (for example, a user computer having a graphical user interface or a web browser, through which the user may interact with the implementations of the systems and the technologies described herein), or a computing system that includes any combination of such backend components, middleware components, or frontend components. The components of the system may be interconnected by any form or medium of digital data communication (e.g., communication network) . Examples of the communication network include: local area network (LAN), wide area network (WAN), the Internet, and block chain network.

The computer system may include a client and a server. The client and the server are generally far from each other and usually interact through the communication network. The relationship between the client and the server is generated by computer programs that run on the corresponding computer and have a client-server relationship with each other.

According to the technical solution of the embodiments of the present disclosure, first acquires a medical quality control requirement of a user, and converts the medical quality control requirement into structured data corresponding thereto; then detects, through a pre-established rule-based reasoning network, whether medical entities in the structured data meet detection conditions corresponding to a leaf node, with each field in the structured data as a medical entity; determines, in response to the medical entities in the structured data meeting the detection conditions corresponding to the leaf node, that the medical quality control requirement hits the leaf node; and pushes medical conflict information corresponding to the leaf node to the user. That is, embodiments of the present disclosure may pre-establish a rule-based reasoning network. After converting the medical quality control requirement into the corresponding structured data, it may detect whether the medical entities in the structured data meet the detection conditions corresponding to a leaf node through the rule-based reasoning network. Therefore, it may detect whether there is the medical conflict information in the medical quality control requirement, and achieve the purpose of promptly reminding the user of a medical conflict. However, in the existing medical conflict detection methods, independent rule fragments are used to restrict the relationships between medical entities. If there is a side relationship between the medical entities, the existing technical solutions cannot use the side relationship information of the medical entities; since the rule fragments are completely independent, a potential conflict relationship between the medical entities cannot be detected using the existing technical solutions. Because an embodiment of the present disclosure adopts the technical means of pre-establishing a rule-based reasoning network, it overcomes the technical problem of the inability to comprehensively and accurately detect a medical conflict in the prior art, and achieves the technical effects of more comprehensive and accurate detection of a medical conflict, more convenient expansion of medical data, and user-friendly; moreover, the technical solution of the embodiments of the present disclosure is simple and convenient to implement, easy to popularize, and have a wider application range.

It should be understood that the various forms of processes shown above may be used to reorder, add, or delete steps. For example, the steps described in embodiments of the present disclosure may be performed in parallel, sequentially, or in different orders, as long as the desired results of the technical solution disclosed in embodiments of the present disclosure can be achieved, no limitation is made herein.

The above specific embodiments do not constitute limitation on the protection scope of the present disclosure. Those skilled in the art should understand that various modifications, combinations, sub-combinations and substitutions may be made according to design requirements and other factors. Any modification, equivalent replacement and improvement, etc. made within the spirit and principle of the present disclosure shall be included in the protection scope of the present disclosure.

## Claims

1. A method for detecting a medical conflict, the method comprising:
acquiring (S101) a medical quality control requirement of a user, and converting the medical quality control requirement into structured data corresponding thereto;
detecting (S102), through a pre-established rule-based reasoning network, whether medical entities in the structured data meet detection conditions corresponding to a leaf node, with each field in the structured data as a medical entity;
in response to the medical entities in the structured data meeting the detection conditions corresponding to the leaf node, determining (S103) that the medical quality control requirement hits the leaf node; and
pushing (S104) medical conflict information corresponding to the leaf node to the user.

2. The method according to claim 1, wherein the detecting (S102), through a pre-established rule-based reasoning network, whether medical entities in the structured data meets detection conditions corresponding to a leaf node, comprises:
extracting (S203) a medical entity from the structured data as a current medical entity, flowing the current medical entity from a root node of the rule-based reasoning network to a node in a penultimate layer of nodes, and using the node in the penultimate layer of nodes, to which the current medical entity flows, as a minimum unit node hit by the current medical entity; and repeatedly performing the operation of extracting a current medical entity, until each medical entity in the structured data flows to a minimum unit node hit by the each medical entity; and
detecting (S204) whether the medical entities in the structured data meet the detection conditions corresponding to a leaf node, based on the minimum unit nodes hit by the medical entities in the structured data.

3. The method according to claim 2, wherein the detecting (S204) whether the medical entities in the structured data meets the detection conditions corresponding to a leaf node, based on the minimum unit nodes hit by the medical entities in the structured data, comprises:
in response to the minimum unit nodes hit by the medical entities in the structured data correspond to one leaf node of the rule-based reasoning network, determining that the medical entities in the structured data meet the detection conditions corresponding to the leaf node; and
in response to the minimum unit nodes hit by the medical entities in the structured data correspond to a plurality of leaf nodes of the rule-based reasoning network, determining that medical entities in the structured data do not meet the detection conditions corresponding to a leaf node.

4. The method according to claim 2, wherein the flowing (S203) the current medical entity from the root node of the rule-based reasoning network to a node in a penultimate layer of nodes, comprises:
using the root node as the node hit by the current medical entity; and
determining, on the node hit by the current medical entity, whether the current medical entity meets a detection condition corresponding to a node in a next layer of nodes in the rule-based reasoning network; in response to the current medical entity meeting a detection condition corresponding to the node in the next layer of nodes, using the node in the next layer of nodes as the node hit by the current medical entity; and repeatedly performing the operation, until the current medical entity flows from the root node to a node in the penultimate layer of nodes.

5. The method according to claim 1, wherein after the acquiring (S101) the medical quality control requirement of the user, and before the converting the medical quality control requirement into the structured data corresponding thereto, the method further comprises:
extracting a key field from the medical quality control requirement; determining a supplementary field associated with the key field based on the extracted key field;
searching medical information corresponding to the key field and medical information corresponding to the supplementary field in a pre-stored medical database; and
in response to the medical information corresponding to the key field and the medical information corresponding to the supplementary field being found in the medical database, performing the operation of converting the medical quality control requirement into the structured data corresponding thereto, based on the key field and the medical information corresponding to the key field, and the supplementary field and the medical information corresponding to the supplementary field.

6. An apparatus (500) for detecting a medical conflict, the apparatus comprising: a conversion module (501), a detection module (502), a determination module (503) and a pushing module (504); wherein,
the conversion module (501), is configured to acquire (S101) a medical quality control requirement of a user, and convert the medical quality control requirement into structured data corresponding thereto;
the detection module (502), is configured to detect (S102), through a pre-established rule-based reasoning network, whether medical entities in the structured data meet detection conditions corresponding to a leaf node, with each field in the structured data as a medical entity;
the determination module (503), is configured to determine (S103), in response to the medical entities in the structured data meeting the detection conditions corresponding to the leaf node, that the medical quality control requirement hits the leaf node; and
the pushing module (504), is configured to push (S104) medical conflict information corresponding to the leaf node to the user.

7. The apparatus according to claim 6, wherein the detection module (502) comprises: a flow submodule (5021) and a detection submodule (5022); wherein,
the flow submodule (5021) is configured to extract (S203) a medical entity from the structured data as a current medical entity, flow the current medical entity from a root node of the rule-based reasoning network to a node in a penultimate layer of nodes, and use the node in the penultimate layer of nodes, to which the current medical entity flows, as a minimum unit node hit by the current medical entity; and repeatedly perform the operation of extracting a current medical entity, until each medical entity in the structured data flows to a minimum unit node hit by the each medical entity; and
the detection submodule (5022) is configured to detect (S204) whether the medical entities in the structured data meet the detection conditions corresponding to a leaf node, based on the minimum unit nodes hit by the medical entities in the structured data.

8. The apparatus according to claim 7, wherein:
the detection submodule (502), is specifically configured to determine, in response to the minimum unit nodes hit by the medical entities in the structured data correspond to one leaf node of the rule-based reasoning network, that the medical entities in the structured data meet the detection conditions corresponding to the leaf node; and determine, in response to the minimum unit nodes hit by the medical entities in the structured data correspond to a plurality of leaf nodes of the rule-based reasoning network, that the medical entities in the structured data do not meet the detection conditions corresponding to a leaf node.

9. The apparatus according to claim 7, wherein:
the flow submodule (5021), is specifically configured to use the root node as the node hit by the current medical entity; and determine, on the node hit by the current medical entity, whether the current medical entity meets a detection condition corresponding to a node in a next layer of nodes in the rule-based reasoning network; in response to the current medical entity meeting a detection condition corresponding to the node in the next layer of nodes, use the node in the next layer of nodes as the node hit by the current medical entity; and repeatedly perform the operation, until the current medical entity flows from the root node to a node in the penultimate layer of nodes.

10. The apparatus according to claim 6, wherein the conversion module (501), is further configured to extract a key field from the medical quality control requirement; determine a supplementary field associated with the key field based on the extracted key field; search medical information corresponding to the key field and medical information corresponding to the supplementary field in a pre-stored medical database; and perform the operation of converting the medical quality control, in response to the medical information corresponding to the key field and the medical information corresponding to the supplementary field being found in the medical database, requirement into corresponding structured data, based on the key field and the medical information corresponding to the key field, and the supplementary field and the medical information corresponding to the supplementary field.

11. An electronic device, comprising:
at least one processor (701); and
a memory (702), communicatively connected to the at least one processor (701); wherein,
the memory (702), storing instructions executable by the at least one processor (701), the instructions, when executed by the at least one processor (701), cause the at least one processor (701) to perform the method according to any one of claims 1-5.

12. Anon-transitory computer readable storage medium, storing computer instructions, the computer instructions, being used to cause a computer to perform the method according to any one of claims 1-5.
